# EUROPÄISCHE PATENTSCHRIFT

(11) **EP 0 783 013 B1**
(45) Veröffentlichungstag und Bekanntmachung des Hinweises auf die Patenterteilung: **19.09.2001**
(21) Anmeldenummer: 96118806.7
(22) Anmeldetag: 23.11.1996
(51) Int. Cl.: C08G 69/10, C08G 73/06

(54) **Copolymere Polyaminosäureester**
Copolymers containing amino acid ester units
Copolymères d'ester de polyacides aminés

(30) Priorität: 07.12.1995 DE 19545678
(43) Veröffentlichungstag der Anmeldung: 09.07.1997
(73) Patentinhaber: Goldschmidt AG, 45127 Essen (DE)
(72) Erfinder: Grüning, Burghard, Dr., 45134 Essen (DE); Rau, Harald, 45139 Essen (DE); Simpelkamp, Jörg, Dr., 45130 Essen (DE); Weitemeyer, Christian, Dr., 45134 Essen (DE)

(56) Entgegenhaltungen:
- WO-A-94/01486
- DE-A- 2 253 190
- US-A- 3 371 069
- US-A- 3 819 588
- US-A- 4 450 150
- US-A- 4 675 381
- US-A- 5 118 784
- US-A- 5 292 858

## Beschreibung

Polyaminosäurederivate, nämlich Polyasparaginsäure, haben in jüngster Zeit aufgrund ihrer Eigenschaften besondere Aufmerksamkeit gefunden. Ein Vorteil des Polyasparaginsäuregerüstes ist die sehr gute Umweltverträglichkeit sowie die biologische Abbaubarkeit. Die biologische Abbaubarkeit der polymeren Asparaginsäuren ist ein Resultat der naturnahen, von Polyaminosäuren abgeleiteten Grundstruktur. Die vorteilhaften Eigenschaften von Polyasparaginsäuren bezüglich der biologischen Abbaubarkeit im Vergleich zu Polymeren mit C-C-Gerüst (z. B. Polyacrylaten) sind beschrieben worden (Abstracts of papers of the ACS, 1994, V208, 423-4; MB Freeman, YH Paik, G Swift, R Wilczynski, SK Wolk, KM Yocom). Es werden vorwiegend Anwendungen als biologisch abbaubare Komplexierungsmittel, Enthärter und Waschmittel-Builder vorgeschlagen.

Unmittelbare Synthesevorstufe ist in den meisten Fällen Polysuccinimid, das cyclische Imid der Polyasparaginsäure oder Derivate monoethylenisch ungesättigter Carbonsäuren, wie Maleinsäure.

So wird in EP-A-0 578 449 die Synthese von Polysuccinimid durch Erhitzen von Asparaginsäure, in Polyalkylenglykolen, allein oder im Gemisch mit weiteren Aminosäuren, beschrieben. WO 92/14753 beschreibt die Synthese von Polysuccinimid und Polyasparaginsäure durch thermische Kondensation von Asparaginsäure. US-A 5 219 952 beschreibt die Synthese von Polysuccinimid aus Maleinsäureanhydrid und Ammoniak sowie die Hydrolyse des Produktes zu Polyasparaginsäure. EP-A-0 578 448 beschreibt die Synthese von Polysuccinimid durch Erhitzen von Aminosäuren und monoethylenisch ungesättigten Dicarbonsäuren bzw. deren Ammoniumsalzen.

Diese Verbindungen zeigen zahlreiche vorteilhafte Eigenschaften, besitzen jedoch keine oberflächenaktiven Eigenschaften. Um Verbindungen zu erhalten, die die positiven Eigenschaften der Polyasparaginsäure mit tensidischen Eigenschaften verbinden, ist die Einführung von hydrophoben, ölverträglichen Molekülteilen in das überwiegend hydrophile Polyasparaginsäuregerüst erforderlich.

Die Umsetzung von Polysuccinimid mit Aminen zu Polyasparaginsäureamiden ist ebenfalls bekannter Stand der Technik (z. B. DE-A 22 53 190). In US-A 5 292 858 wird die Synthese von copolymeren Polyaminosäureamiden durch Hydrolyse von Polysuccinimidderivaten, hergestellt durch Umsetzung von Maleinsäurehalbestern mit Ammoniak oder Aminen, beschrieben.

Nach diesen Vorschriften sind Copolymere mit freien Carbonsäuregruppierungen und Alkylamidgruppen zugänglich. Diese Copolymere weisen jedoch gravierende Nachteile auf. So enthalten die Produkte herstellungsbedingt in mehr oder weniger geringen Mengen freie Alkylamine, die in vielen Anwendungen unerwünscht sind und deren Verwendung auch toxikologische und ökologische Nachteile mit sich bringen kann. Die Alkylamine können zusätzlich auch durch z. B. hydrolytische Spaltung während des Gebrauches derartiger Copolymere freigesetzt werden.

Die WO 94/01486 beschreibt modifizierte Polyasparaginsäuren, erhältlich durch Polykondensation von (a) 1 bis 99,9 mol-%, Asparaginsäure mit (b) 99 bis 0,1 mol-% an Fettsäuren, mehrbasischen Carbonsäuren, Anhydriden mehrbasischen Carbonsäuren, mehrbasische Hydroxycarbonsäuren, einbasische Polyhydroxycarbonsäuren, Alkoholen, Aminen, alkoxylierten Alkoholen und Aminen, Aminozuckern, Kohlenhydraten, Zuckercarbonsäuren und/oder nichtproteinogenen Aminocarbonsäuren oder durch Polymerisieren von monoethylenisch ungesättigten Monomeren in Gegenwart von Polyasparaginsäuren nach Art einer radikalisch initiierten Pfropfcopolymerisation, Verfahren zur Herstellung der modifizierten Polyasparaginsäuren sowie Verwendung der modifizierten Polyasparaginsäuren als Zusatz zu Wasch- und Reinigungsmitteln, als Wasserbehandlungsmittel und als Belagsverhinderer bei der Eindampfung von Zuckersaft.

Copolymere Polyasparaginsäurederivate, in denen ein Teil der Carboxylgruppen mit Alkoholen, insbesondere mit Fettalkoholen, verestert sind, sind bislang nicht bekannt. Derartige neue Esterderivate werden durch die Erfindung bereitgestellt. Da die Herstellung der erfindungsgemäßen Verbindungen nicht auf der Verwendung von Aminen basiert und Amine auch nicht in gebundener Form in den Copolymeren enthalten sind, weisen die erfin dungsgemäßen Verbindungen die oben genannten Nachteile nicht auf.

Ein weiterer Vorteil der erfindungsgemäßen Copolymeren ist ihre sehr gute Umweltverträglichkeit, die sich aus der naturnahen, von Polyaminosäuren abgeleiteten Grundstruktur sowie der Anbindung der Alkylseitenketten über Estergruppen ergibt.

Die erfindungsgemäßen Copolymeren können durch die Art und Dichte der Alkylseitenketten ebenso wie durch ihr Molgewicht verschiedenartigsten anwendungstechnischen Anforderungen angepaßt werden. So können sich kurzkettig alkylsubstituierte Derivate als Sequestriermittel eignen oder in korrosionsschützenden Anstrichen Verwendung finden, zumal sie auch in polaren organischen Lösungsmitteln löslich sein können.

Langkettig alkylsubstituierte Copolymere zeigen grenzflächenaktive Eigenschaften. Durch Variation verschiedener Parameter, wie Kettenlänge der langkettigen Komponente, Polymerisationsgrad des Copolymeren, Verhältnis hydrophober Seitenketten zu freien Carbonsäuregruppen etc. sind umweltverträgliche oberflächenaktive Produkte mit herausragenden Anwendungseigenschaften erhältlich. Diese sind vielseitig einsetzbar z. B. als W/O- wie auch als O/W-Emulgatoren. Die schaumstabilisierenden Eigenschaften ermöglichen eine Verwendung als Schaumverstärker z. B. in milden kosmetischen Reinigungsmitteln oder in Haushaltsdetergentien. Weitere Anwendungsgebiete der oberflächenaktiven Materialien mit der für Polyasparaginsäuren charakteristischen Komplexierungsfähigkeit für zweiwertige Metallionen wie Ca²⁺ sind Waschmittelhilfsstoffe und Builder, Dispergierhilfsmittel oder Konditioniermittel für kosmetische Anwendungen.

Die erfindungsgemäßen von Polyasparaginsäure abgeleiteten Copolymeren bestehen zu mindestens 75 % der vorhandenen Einheiten aus Struktureinheiten der allgemeinen Formeln (I) und (II) 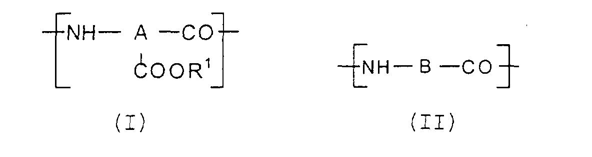 in der die Strukturelemente A gleiche oder verschiedene trifunktionelle Kohlenwasserstoffradikale mit 2 C-Atomen der Struktur (A1) oder (A2) sind 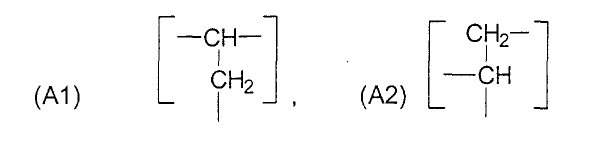 wobei ein Copolymeres aus mindestens drei Einheiten der Formel (I) besteht, worin
- R¹: die Bedeutung von R², R³ und R⁴ haben kann, wobei
R² ein oder mehrere Reste aus der Gruppe der Alkali-, Erdalkalimetalle, Wasserstoff oder Ammonium, [NR⁵R⁶R⁷R⁸]⁺ sind, worin R⁵ bis R⁸ unabhängig voneinander Wasserstoff, Alkyl oder Hydroxyalkyl bedeuten,
R³ gleiche oder verschiedene, geradkettige oder verzweigte, gesättigte oder ungesättigte Alkylreste R⁹ mit 6 bis 24 C-Atomen oder Radikale der Struktur -X-R⁹ sind, wobei X eine Oligo- oder Polyoxyalkylenkette mit 1 bis 100 Oxyalkyleneinheiten ist,
R⁴ gleiche oder verschiedene, geradkettige oder verzweigte, gesättigte oder ungesättigte Alkylreste mit 1 bis 5 C-Atomen sind, und mindestens jeweils ein Rest R¹ die Bedeutung von R² und mindestens ein Rest R¹ die von R³ oder R⁴ annehmen muß und die Einheiten [-NH-B-CO] Bausteine aus der Gruppe der proteinogenen oder nicht proteinogenen Aminosäuren sind und zu nicht mehr als 20 Gew.-% enthalten sind.

Als Aminosäurebausteine [-NH-B-CO] aus der Gruppe der proteinogenen Aminosäuren kommen z. B. Glutamin, Asparagin, Lysin, Alanin, Glycin, Tyrosin, Tryptophan, Serin und Cystein sowie deren Derivate in Frage; nicht proteinogene Aminosäuren können z. B. β-Alanin, ω-Amino-1-alkansäuren etc. sein.

Erfindungsgemäß sind Verbindungen, bei denen mindestens eine freie Carboxylatgruppe (R¹ = H, Metall, Ammonium) vorhanden ist, ein Rest R⁴ aus der Gruppe der geradkettigen oder verzweigten, gesättigten oder ungesättigten Alkylreste mit 1 bis 5 C-Atomen stammt (z. B. Methyl, Ethyl, n-Propyl, i-Propyl, n-Butyl, i-Butyl, s-Butyl, n-Pentyl) und ein Rest R³ gleiche oder verschiedene Radikale der Struktur R⁹-X- sind, wobei R⁹ aus der Gruppe der geradkettigen oder verzweigten, gesättigten oder ungesättigten Alkylreste mit 6 bis 24 C-Atomen stammt (z. B. verzweigte oder lineare Octyl-, Decyl-, Dodecyl-, Tetradecyl-, Hexadecyl-, Octadecyl-, Docosylreste, auch ungesättigte und mehrfach ungesättigte Spezies wie z. B. Oleyl) und X eine Polyoxyalkylenkette von 0 bis 100 Alkylenglykoleinheiten, vorzugsweise abgeleitet von Ethylenoxid, Propylenoxid oder Gemischen daraus. Eine bevorzugte Form der erfindungsgemäßen Copolymeren enthält Alkylreste R⁹ mit 8 bis 24 C-Atomen ohne Alkylenglykolspacer (Alkylenglykolkettenlänge 0).

Die Herstellung der Copolymeren kann durch sequentielle Umsetzung von Polyasparaginsäureimid (Polysuccinimid, hergestellt beispielsweise nach US-A 5 219 952) mit Alkoholen R⁴OH in einer ersten Stufe und gegebenenfalls nachfolgende partielle Reaktion mit Alkoholen R³OH erfolgen. Dabei haben R³ und R⁴ die oben angegebene Bedeutung. Vorzugsweise ist R⁴ ein geradkettiger oder verzweigter Alkylrest mit 1 bis 4 C-Atomen. Vorzugsweise ist R³ ein geradkettiger oder verzweigter, gesättigter oder ungesättigter Alkylrest mit 8 bis 24 C-Atomen. Bei Verwendung von Polyoxyalkylenketten X sind diese vorzugsweise abgeleitet von Ethylenoxid, Propylenoxid oder Gemischen daraus. In einer dritten Stufe können die von R⁴OH abgeleiteten Estergruppen unter milden Bedingungen teilweise oder vollständig hydrolysiert werden, unter Freisetzung der für die erfindungsgemäßen Copolymeren charakteristischen Carbonsäure- bzw. Carboxylatgruppen. Dabei bleiben die die erfindungsgemäßen Copolymeren weiterhin kennzeichnenden Estergruppen der langkettigen Alkohole bevorzugt erhalten.

Als Alkohole der Formel R⁴OH können beispielsweise Verbindungen mit R⁴ = Methyl, Ethyl, n-Propyl, i-Propyl, n-Butyl, i-Butyl, s-Butyl und n-Pentyl eingesetzt werden. Besonders bevorzugt werden Methyl- und Ethylalkohol verwendet.

Als Rest R³ können z. B. lineare oder verzweigte Decyl-, Dodecyl-, Tetradecyl-, Hexadecyl-, Octadecyl-, Docosylreste oder ungesättigte Alkylreste wie Oleyl eingesetzt werden.

Ein bevorzugtes Verfahren erfolgt durch Umsetzung des Asparaginsäureimides mit einem Überschuß des Alkohols bei 1 bis 20 bar und 65 bis 200 °C für 6 bis 48 h, mit oder ohne Zusatz weiterer Lösungsmittel, wie Dimethylsulfoxid, Dimethylformamid, Ethylenglykol, Oligoethylenglykole, Mono- und Oligoethylenglykolalkylether mit oder ohne Anwesenheit eines sauren oder basischen Katalysators, vorzugsweise Mineralsäure, organische Säuren, saure Ionenaustauscher, Alkali- und Erdalkalisalze organischer und anorganischer Säuren, Alkali- und Erdalkalihydroxide, Alkoxylate, besonders vorzugsweise Alkalioder Erdalkalialkoxylate der eingesetzten Alkohole.

Ein weiteres Verfahren zur Herstellung der erfindungsgemäßen Copolymeren ist dadurch gekennzeichnet, daß man ein Gemisch von Monoestern monoethylenisch ungesättigter Dicarbonsäuren mit Ammoniak umsetzt bzw. die Ammoniumsalze dieser Säuren thermisch in das Polymer überführt. Eingesetzt werden können Derivate der Maleinsäure, Fumarsäure, Itaconsäure, Alkenylbernsteinsäure, Alkylmaleinsäure, Citraconsäure oder deren Ammoniumsalze, vorzugsweise Derivate der Maleinsäure, Fumarsäure oder Itaconsäure, besonders vorzugsweise Maleinsäurederivate der allgemeinen Formeln (III) und (IV) 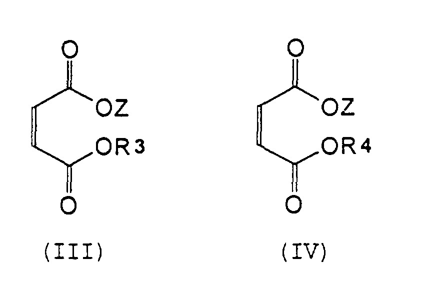 wobei Z für Wasserstoff und/oder Ammonium, R³ und R⁴ für die oben genannten Reste stehen. Diese Maleinsäurederivate können jeweils alleine oder im Gemisch miteinander eingesetzt werden.

Vorzugsweise eingesetzte Reste R³ sind Alkylreste mit 8 bis 24 C-Atomen ohne Polyalkylenglykolanteil, beispielsweise lineare oder verzweigte Decyl-, Dodecyl-, Tetradecyl-, Hexadecyl-, Octadecyl- oder Docosylreste sowie ungesättigte Alkylreste, wie z. B. Oleyl. Vorzugsweise eingesetzte Reste R⁴ sind Alkylreste mit 1 bis 4 C-Atomen ohne Polyalkylenglykolanteil, vorzugsweise Methyl, Ethyl, n-Propyl, i-Propyl, n-Butyl, i-Butyl, s-Butyl. Das Verhältnis der Komponenten (III) und (IV) beträgt vorzugsweise zwischen 100 : 0 und 10 : 90, besonders vorzugsweise zwischen 75 : 25 und 25 : 75.

Im erfindungsgemäßen Verfahren werden die eingesetzten Maleinsäuremonoester mit 0,5 bis 1,5, vorzugsweise 0,8 bis 1,5 Equivalenten Ammoniak (als Ammoniakgas bzw. als Lösung) umgesetzt. Die Reaktion kann mit oder ohne Zusatz eines organischen Lösungsmittels erfolgen. Als Lösungsmittel kommen beispielsweise Alkohole, Ether, Oligo- und Poly(alkylen)glykole bzw. -glykolether, Dimethylsulfoxid, Dimethylformamid in Frage. Bei Verwendung eines Lösungsmittels wird vorzugsweise der kurzkettige Alkohol R⁴OH eingesetzt. Die Umsetzung erfolgt bei Temperaturen von 0 bis 150 °C, vorzugsweise 40 bis 120 °C. Eine bevorzugte Vorgehensweise ist beispielsweise die Umsetzung von Maleinsäuremonoalkylester und wäßrigem oder gasförmigem Ammoniak bei 50 bis 70 °C, Entfernen des gegebenenfalls aus der Ammoniaklösung stammenden Wassers bzw. des Reaktionswassers bei vermindertem Druck und 20 bis 120 °C, vorzugsweise bei 50 bis 80 °C, sowie, weiterhin unter vermindertem Druck, langsame Steigerung der Temperatur auf 90 bis 150 °C, vorzugsweise auf 100 bis 120 °C, unter Rühren der zunehmend viskoser werdenden Reaktionsmischung. Dabei findet die Umsetzung zum Copolymeren statt. Unter den Reaktionsbedingungen werden gleichzeitig ein Teil der Estergruppen, bevorzugt die von R⁴OH abgeleiteten, hydrolysiert und die gewünschten Carbonsäure- bzw. Carboxylatgruppen freigesetzt. Unter den Reaktionsbedingungen tritt eine Bildung von cyclischen Imidstrukturen (V) aus den hydrolysierten Asparaginsäureeinheiten (VI) nur untergeordnet auf.

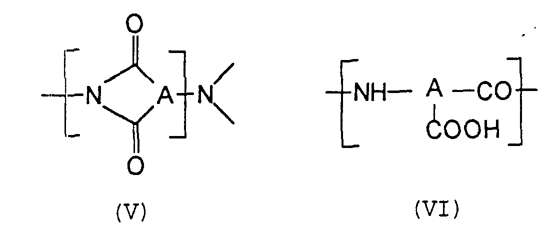

Darüber hinaus ist anzunehmen, daß im Produkt vorhandene Imidstrukturen (V) zudem unter Anwendungsbedingungen in Gegenwart eines wäßrigen Mediums unter Freisetzung der Säureeinheiten (VI) geöffnet werden.

Durch milde partielle oder vollständige Hydrolyse, bevorzugt der vom kurzkettigen Alkohol R⁴OH abgeleiteten Esterfunktionen kann, wenn gewünscht, der Anteil freier Säuregruppen bzw. Carboxylatgruppen weiter erhöht werden. Zur Hydrolyse werden Lösungen oder Suspensionen der Copolymeren in organischen Lösungsmitteln mit Wasser oder Wasserdampf umgesetzt, oder die Copolymeren werden in Wasser mit und ohne Zusatz organischer Cosolventien hydrolysiert. Diese Reaktion kann ohne Katalysator oder in Gegenwart von organischen oder anorganischen Säuren, sauren Ionenaustauschern oder sauren Mineralien oder basischen Verbindungen, wie Metallhydroxiden oder Aminen erfolgen. Vorzugsweise als Basen eingesetzt werden Alkali- und Erdalkalimetallhydroxide, z. B. Natriumhydroxid und Kaliumhydroxid, in katalytischen oder stöchiometrischen Mengen.

Durch Zusatz von amino- und carboxyfunktionellen Verbindungen zur Reaktionsmischung können Copolymere erhalten werden, in denen die angebotenen Bausteine über Amidbindungen gebunden vorliegen. Geeignete Bausteine sind Aminosäuren aus der Gruppe der 20 proteinogenen Aminosäuren, welche als Monomere in allen natürlichen Proteinen enthalten sind, in enantiomerenreiner oder racemischer Form, wie z. B. Glutamin, Asparagin, Lysin, Alanin, Glycin, Tyrosin, Tryptophan, Serin und Cystein sowie deren Derivate, oder nicht proteinogenen Aminosäuren mit jeweils einer oder mehreren Amino- bzw. Carboxyfunktionen, wie z. B. β-Alanin, ω-Amino-1-alkansäuren. Die Bausteine, vorzugsweise 0 bis 20 Gew.-% werden der Ausgangsmischung der Maleinsäurederivate zugesetzt oder zur Modifizierung der Kettenenden nach erfolgter Synthese des Polymeren mit diesem umgesetzt, vorzugsweise unter Zusatz polarer Solventien, wie z. B. Alkoholen oder Dimethylformamid.

Die erfindungsgemäßen Copolymeren zeigen hervorragende Eigenschaften als Sequestriermittel, als Additive zu Farben und Lacken sowie insbesondere bei der Stabilisierung von O/W- und W/O-Emulsionen und als Schaumstabilisatoren. Aufgrund ihrer von Polyaminosäuren abgeleiteten Struktur besitzen sie hohe Umweltverträglichkeit.

Beispiele für erfindungsgemäße Copolymere sind:

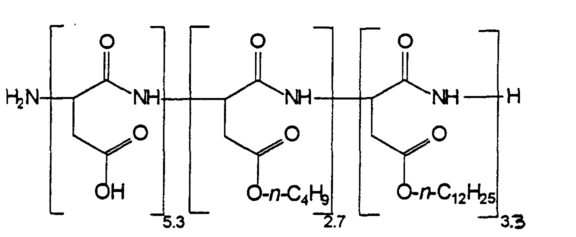

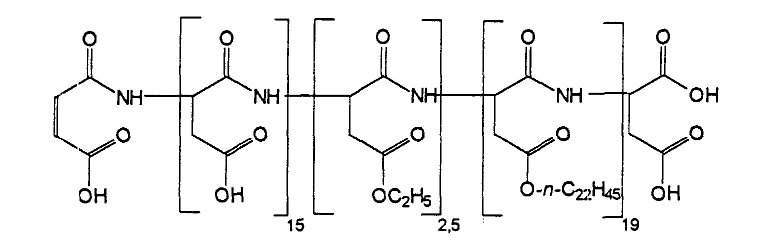

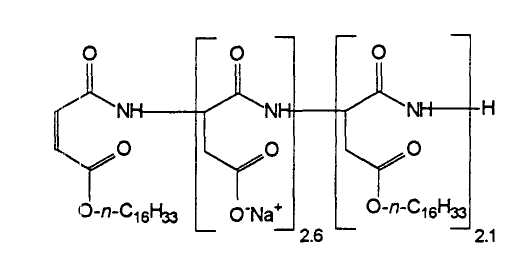

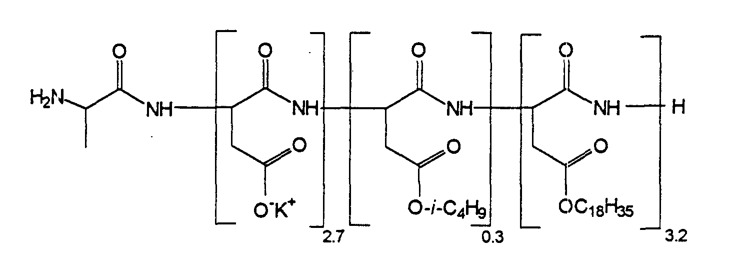

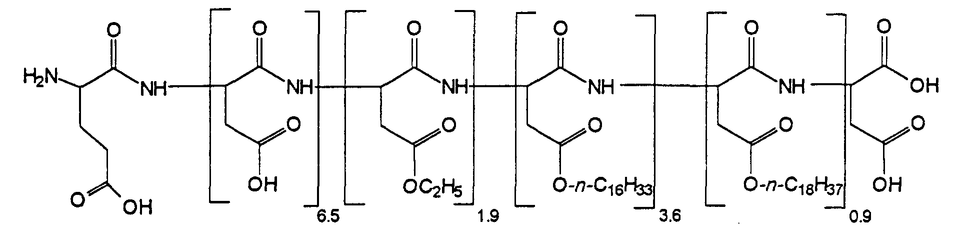

Zur Vereinfachung sind alle beispielhaften Strukturen in α-Verknüpfung (VII) dargestellt; dieses bedeutet keine Aussage über das tatsächliche Verhältnis an α/β-Strukturen in den erfindungsgemäßen Polymeren.

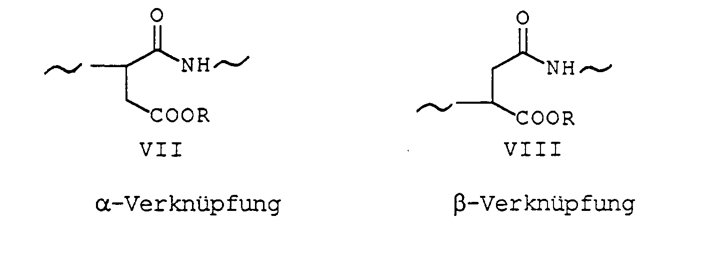

### Beispiel 1

In einem 2000-ml-Kolben mit einem scherkraftreichen Rührer, Thermometer und Destillationsaufsatz werden 710 g Maleinsäuremonododecylester (2,5 Mol), 360 g Maleinsäuremonoethylester (2,5 Mol) und 100 ml n-Butanol auf 50 °C erwärmt. Innerhalb von 20 Min. werden 340 g wäßriger Ammoniaklösung 25 %ig (5 Mol) bei einer max. Temperatur von 70 °C zugetropft. Nach einer Reaktionszeit von 30 Min. wird unter langsamer Druckverminderung auf 30 mbar und einer max. Temperatur von 80 °C Wasser und Butanol innerhalb von 2 Stunden abdestilliert. Sobald kein Wasser mehr sichtbar im Kühler kondensiert, wird der Ansatz im Vakuum innerhalb von 6 h langsam auf 110 °C erhitzt. Es liegt danach eine gelbbraune, karamellartige Masse vor. Nach ¹³C-NMR liegt eine partiell veresterte Polyasparaginsäure vor, bei der das Verhältnis -COOH : -COOC₁₂H₂₅ ca. 1 : 1 ist. Der Anteil des Ethylesters und des Dodecylalkohols liegen unter 1 Mol-%. Das Gelchromatogramm gemessen in Lösung in Tetrahydrofuran zeigt ein mittleres Molgewicht von ca. 900 an (entsprechend einer Kettenlänge von N = ca. 4,5; Eichung gegen Polystyrol).

### Beispiel 2

500 g des Produktes aus Beispiel 1 werden in einem evakuierbaren Kneter auf 100 °C aufgeheizt und innerhalb von 6 h bei 30 mbar langsam auf 120 °C erhitzt. Das resultierende Produkt hat nach GPC ein mittleres Molekulargewicht von ca. 2400 (N = ca. 12).

### Beispiel 3

Wie unter Beispiel 1 beschrieben, werden 442 g Maleinsäuremonostearylester (1,2 Mol), 826 g Maleinsäuremonobutylester (4,8 Mol), 100 ml n-Butanol und 408 g Ammoniaklösung 25 %ig (6 Mol) zur Reaktion gebracht. Das resultierende, feste, leicht klebrige Produkt weist nach ¹³C-NMR ein Verhältnis der Gruppen -COOH : -COOC₄H₉ : -COOC₁₈H₃₇ von ca. 2,8 : 1,2 : 1 auf. Das GPC zeigt ein mittleres Molgewicht von ca. 750 (N = ca. 4,3) an.

### Beispiel 4

500 g des Produktes aus Beispiel 3 werden wie unter Beispiel 2 beschrieben zur Erhöhung des Molgewichtes weiterverarbeitet. Das resultierende feste Produkt hat nach GPC ein mittleres Molgewicht von ca. 2000 (N = ca. 11,5).

### Beispiel 5

Wie unter Beispiel 1 beschrieben, werden 1098 g Maleinsäuremonooleylester (3 Mol), 316 g Itaconsäuremonoethylester (2 Mol) und 100 ml n-Butanol mit 340 g Ammoniaklösung 25 %ig (5 Mol) zur Reaktion gebracht. Das resultierende hochviskose Produkt weist nach ¹³C-NMR ein Verhältnis der -COOH : -COOC₁₈H₃₅ von ca. 0,7 : 1 auf. Das GPC zeigt ein mittleres Molgewicht von ca. 1100 (N = ca. 4,1) an.

### Beispiel 6

485 g Polyasparaginsäureimid (mittleres MG 1500), 800 g Methanol und 17,8 g Natriummethylat werden in einer Druckapparatur bei 18 bar auf 170 °C erhitzt. Diese Bedingungen werden über 8 h gehalten. Nach dem Abkühlen wird der Rückstand (35 g) abfiltriert und das überschüssige Methanol bis 100 °C und 30 mbar abdestilliert. Das GPC des verbleibenden Rückstandes zeigt ein mittleres MG von ca. 1400. Der Veresterungsgrad beträgt nach ¹³C-NMR 78 %. Das Produkt wird in 2000 g Dimethylformamid gelöst. In einer 4000-ml-Apparatur mit Rührer, Thermometer, Kolonne, Rückflußkühler und Kühlfalle werden unter Stickstoff zu der Lösung des Produktes 535 g Oleylalkohol und 9 g Tetraisopropyltitanat gegeben. Der Ansatz wird bei ca. 300 mbar bis zum Rückfluß des Dimethylformamids erhitzt (ca. 125 °C). Die Leistung der Kolonne wird so eingestellt, daß nur Methanol in der Kühlfalle kondensiert. Nach 5 h Reaktionszeit wird die Kolonne entfernt und das Dimethylformamid bei 30 mbar abdestilliert. Das ¹³C-NMR zeigt einen Umesterungsgrad von 88 % der Theorie. Das Produkt wird mit 400 g Wasser und 5 g Natriumhydroxid für 1 h auf 60 °C erwärmt und anschließend zur Umwandlung des Salzes in die freie Säure über eine Säule mit starksaurem Ionenaustauscher (LEWATIT® SPC 108 der Fa. Bayer) gegeben. Danach werden das Wasser und das entstandene Methanol im Rotationsverdampfer bei ca. 30 mbar abgezogen. Das resultierende Produkt hat ein -COOH : -COOC₁₈H₃₅-Verhältnis von ca. 1 : 0,8 und ein mittleres Molgewicht von 1200 (N = ca. 5,3).

### Beispiel 7

485 g Polyasparaginsäureimid (mittleres MG 1500), 1480 g n-Butanol und 31,1 g Natrium-n-butylat werden in einer Druckapparatur bei 5 bar 10 h auf 180 °C erhitzt. Nach dem Abkühlen wird filtriert und das überschüssige n-Butanol bis 100 °C und 30 mbar abdestilliert. Das GPC des verbleibenden Rückstandes zeigt ein mittleres MG von ca. 1200. Der Veresterungsgrad beträgt nach ¹³C-NMR 69 %.

### Beispiel 8

Wie unter Beispiel 1 beschrieben, werden 710 g Maleinsäuremonododecylester (2,5 Mol), 860 g Maleinsäuremonobutylester (5,0 Mol), 300 ml n-Butanol und 510 g Ammoniaklösung 25 %ig (7,5 Mol) sowie 36 g (0,4 Mol) D,L-Alanin zur Reaktion gebracht. Das resultierende, leicht klebrige Produkt weist nach ¹³C-NMR ein Verhältnis der Gruppen -COOH : -COOC₄H₉ : -COOC₁₂H₂₅ : Ala von ca. 1,6 : 0,5 : 1,0 : 0,15 auf. Das GPC zeigt ein mittleres Molgewicht von ca. 645 (N = ca. 3,7) an.

### Beispiele 9 bis 12

Von den Produkten aus den Beispielen 1, 3, 5 und 6 werden die folgenden Zubereitungen mit Natriumlaurylethersulfat-Lösung (28 %ig in Wasser, nachfolgend NaLES genannt) hergestellt:

| Beispiel | Produkt aus Beispiel | Produkt g | 0,1molare Natronlauge g | NaLES g | Wasser g |
|---|---|---|---|---|---|
| 9 | 1 | 10 | 23 | 40 | 0 |
| 10 | 3 | 10 | 0 | 40 | 23 |
| 11 | 5 | 10 | 0 | 40 | 23 |
| 12 | 6 | 10 | 11 | 40 | 12 |

Von den Zubereitungen werden 0,07 g in 200 ml Wasser gelöst.
Als Vergleich werden 0,1 g einer Zubereitung aus 40 g NaLES und 31,2 g einer acylierten Proteinhydrolysatlösung in 200 ml gelöst. In einem 1000-ml-Meßzylinder werden diese Lösungen mit einer Turbine aufgeschäumt. Danach wird die Abnahme des Schaumvolumens beobachtet. Die Ergebnisse sind in der folgenden Tabelle dargestellt (Werte in ml):

| Zeit | Bsp. 9 | Bsp. 10 | Bsp. 11 | Bsp. 12 | Vergleich |
|---|---|---|---|---|---|
| sofort | 160 | 200 | 170 | 180 | 150 |
| 30 Min. | 150 | 180 | 160 | 170 | 120 |
| 60 Min. | 140 | 180 | 160 | 170 | 120 |
| 3 Std. | 130 | 160 | 150 | 160 | 110 |
| 6 Std. | 120 | 150 | 130 | 150 | 100 |
| 24 Std. | 100 | 120 | 100 | 110 | 70 |

### Beispiel 13

7,5 g des Produktes aus Beispiel 2 werden bei 50 °C in 67,5 g dünnflüssigem Paraffinöl (5° Engler) gelöst. An einem schnelllaufenden Rührer werden 75,0 g Wasser zugegeben. Man erhält eine O/W-Emulsion, die über einen Prüfzeitraum von 3 Wochen stabil ist.

### Beispiel 14

15,0 g des Produktes aus Beispiel 4 werden mit 100,0 g Wasser gerührt. Dabei wird durch langsames Zugeben von 29 ml verdünnter Natronlauge (0,1 Mol/l) ein pH-Wert von 7 eingestellt. Der resultierenden, leicht trüben Lösung werden an einem schnelllaufenden Rührer 114 g Paraffinöl (5° Engler) zugesetzt. Die resultierende W/O-Emulsion ist nach 3 Wochen noch stabil.

### Beispiel 15

Das Beispiel 13 wird abgeändert, indem statt Paraffinöl Isopropylmyristat eingesetzt wird. Die erhaltene O/W-Emulsion ist nach 3 Wochen noch stabil.

## Patentansprüche

1. Von Polyasparaginsäuren abgeleitete Copolymere, die zu mindestens 75 % der vorhandenen Einheiten aus Struktureinheiten der allgemeinen Formeln (I) und (II) 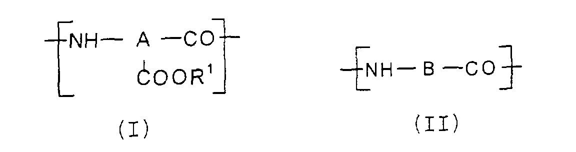 bestehen, in denen die Strukturelemente A gleiche oder verschiedene trifunktionelle Kohlenwasserstoffradikale mit 2 C-Atomen, der Struktur (A1) oder (A2) ist 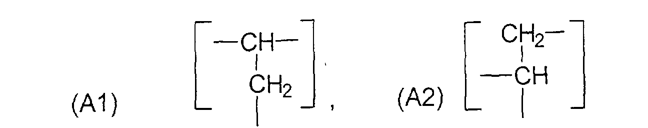 wobei ein Copolymeres aus mindestens drei Einheiten der Formel (I) besteht, worin
R¹ die Bedeutung von R², R³ oder R⁴ haben kann, wobei
R² ein oder mehrere Reste aus der Gruppe der Alkali-, Erdalkalimetalle, Wasserstoff oder Ammonium, [NR⁵R⁶R⁷R⁸] + sind, worin R⁵ bis R⁸ unabhängig voneinander Wasserstoff, Alkyl oder Hydroxyalkyl bedeuten,
R³ gleiche oder verschiedene, geradkettige oder verzweigte, gesättigte oder ungesättigte Alkylreste R⁹ mit 6 bis 24 C-Atomen oder Radikale der Struktur -X-R⁹ sind, wobei X eine Oligo- oder Polyoxyalkylenkette mit 1 bis 100 Oxyalkyleneinheiten ist,
R⁴ gleiche oder verschiedene, geradkettige oder verzweigte, gesättigte oder ungesättigte Alkylreste mit 1 bis 5 C-Atomen sind,
und mindestens jeweils ein Rest R¹ die Bedeutung von R² und mindestens ein Rest R¹ die von R³ oder R⁴ annehmen muß und
die Einheiten [-NH-B-CO] Bausteine aus der Gruppe der proteinogenen oder nicht proteinogenen Aminosäuren sind und zu nicht mehr als 20 Gew.-% enthalten sind.

2. Copolymere nach Anspruch 1, in denen mindestens ein Rest R¹ die Bedeutung von R² und mindestens ein Rest R¹ die Bedeutung von R⁴ hat.

3. Copolymere nach Anspruch 1, in denen mindestens ein Rest R¹ die Bedeutung von R² und mindestens ein Rest R¹ die Bedeutung von R³ hat.

4. Copolymere nach Anspruch 1, in denen mindestens ein Rest R¹ die Bedeutung von R², mindestens ein Rest R¹ die Bedeutung von R³ und mindestens ein Rest R¹ die Bedeutung von R⁴ hat.

5. Copolymere nach den Ansprüchen 1, 3 und 4, in denen R³ gleiche oder verschiedene, geradkettige oder verzweigte, gesättigte oder ungesättigte Alkylreste R¹⁰ mit 8 bis 24 C-Atomen sind.

6. Verfahren zur Herstellung der Copolymeren gemäß Anspruch 1,
**dadurch gekennzeichnet, daß** man Polyasparaginsäureimid in einer ersten Stufe mit Alkoholen R⁴OH und gegebenenfalls in einer zweiten Stufe mit Alkoholen der Struktur R³OH oder R³-X-OH partiell umsetzt, wobei R⁴, R³ und X die oben genannte Bedeutung haben, und in einer gegebenenfalls nachfolgenden dritten Stufe die von R⁴OH abgeleiteten Estergruppen unter milden Bedingungen vollständig oder zum Teil hydrolysiert.

7. Verfahren zur Herstellung der Copolymeren gemäß Anspruch 1 bis 5, **dadurch gekennzeichnet, daß** man Ester α,β-ungesättigter Dicarbonsäuren oder deren Ammoniumsalze, der allgemeinen Formeln (III) und (IV) 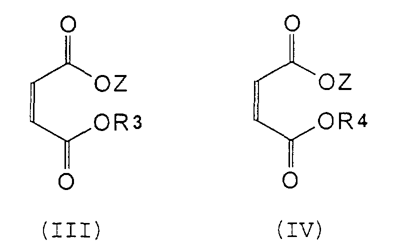 alleine oder im Gemisch miteinander, mit Ammoniak umsetzt und polymerisiert, in denen Z für Wasserstoff und/oder Ammonium steht, und R³ und R⁴ für die oben genannten Reste stehen, gegebenenfalls in Gegenwart von bis zu 20 Gew.-% proteinogener oder nicht proteinogener Aminosäuren oder deren Derivate, sowie gegebenenfalls in weiteren Schritten durch Hydrolyse Gruppen der Struktur der Formel (I), wobei R¹ die Bedeutung von R², mit der oben genannten Definition von R², hat, erzeugt.

8. Verfahren nach Anspruch 7, **dadurch gekennzeichnet, daß** man als Ester α,β-ungesättigte Dicarbonsäuren oder deren Ammoniumsalze und Maleinsäurederivate einsetzt.

9. Verwendung der Copolymeren gemäß den Ansprüchen 1 bis 5 in tensidhaltigen Zubereitungen.

10. Verwendung der Copolymeren gemäß den Ansprüchen 1 bis 5 als Emulgator, Schaumverstärker, Waschmittelhilfsstoff, Komplexagens für mehrwertige Kationen, Dispergierhilfsmittel oder Konditioniermittel.

11. Verwendung der Copolymeren gemäß Anspruch 1 bis 5 als Tensid oder Schaumverstärker in Reinigungsmitteln und kosmetischen Präparaten.

## Claims

1. Copolymers derived from polyaspartic acid and in which at least 75% of the units present consist of structural units of the general formulae (I) and (II) 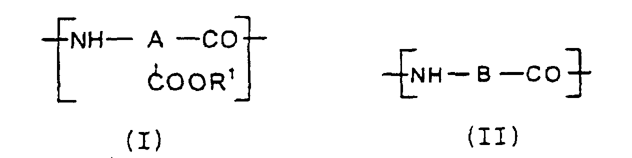 in which the structural elements A are identical or different trifunctional hydrocarbon radicals having 2 carbon atoms of the structure (A1) or (A2) 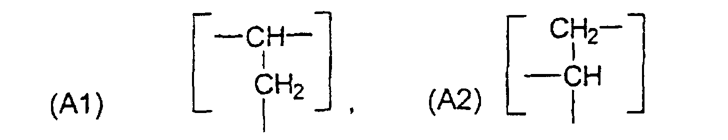 wherein one copolymer consists of at least three units of the formula (I), where
R¹ may be R², R³ or R⁴, of which
R² represents one or more radicals selected from the group consisting of alkali metals, alkaline earth metals, hydrogen and ammonium [NR⁵R⁶R⁷R⁸]⁺, where R⁵ to R⁸ are independently hydrogen, alkyl or hydroxyalkyl,
R³ represents identical or different, straight-chain or branched, saturated or unsaturated alkyl radicals R⁹ of 6 to 24 carbon atoms or radicals of the structure -X-R⁹, where X is an oligo- or polyoxyalkylene chain of 1 to 100 oxyalkylene units,
R⁴ represents identical or different, straight-chain or branched, saturated or unsaturated alkyl radicals of 1 to 5 carbon atoms,
and at least in each case one R¹ is R² and at least one R¹ is R³ or R⁴ and
the [-NH-B-CO] units are building blocks from the group of the proteinogenic or nonproteinogenic amino acids and are present in an amount of not more than 20% by weight.

2. Copolymers according to Claim 1, wherein at least one R¹ is R² and at least one R¹ is R⁴.

3. Copolymers according to Claim 1, wherein at least one R¹ is R² and at least one R¹ is R³.

4. Copolymers according to Claim 1, wherein at least one R¹ is R², at least one R¹ is R³ and at least one R¹ is R⁴.

5. Copolymers according to Claims 1, 3 and 4, wherein R³ represents identical or different, straight-chain or branched, saturated or unsaturated alkyl radicals R¹⁰ of 8 to 24 carbon atoms.

6. Process for preparing the copolymers of Claim 1, **characterized in that** polyaspartic acid imide is partially reacted in a first step with alcohols R⁴OH and optionally in a second step with alcohols of the structure R³OH or R³-X-OH, where R⁴, R³ and X are each as defined above, and in an optionally subsequent third step the ester groups derived from R⁴OH are completely or partially hydrolysed under mild conditions.

7. Process for preparing the copolymers of Claims 1 to 5, **characterized in that** esters of α,β-unsaturated dicarboxylic acids or ammonium salts thereof, of the general formulae (III) and (V) 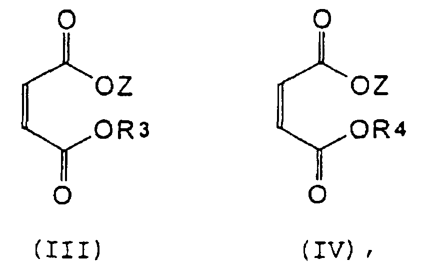 in which Z is hydrogen and/or ammonium and R³ and R⁴ are each as defined above, are alone or in mixture with each other reacted with ammonia and polymerized, optionally in the presence of up to 20% by weight of proteinogenic and/or nonproteinogenic amino acids or derivatives thereof and optionally in further steps groups of the structure of the formula (I) where R¹ is R² as defined above are generated by hydrolysis.

8. Process according to Claim 7, **characterized in that** the esters of α,β-unsaturated dicarboxylic acids or ammonium salts thereof are maleic acid derivatives.

9. Use of the copolymers of Claims 1 to 5 in surfactant preparations.

10. Use of the copolymers of Claims 1 to 5 as an emulsifier, foam booster, laundry detergent auxiliary, complexing agent for polyvalent cations, dispersing aid or conditioner.

11. Use of the copolymers of Claims 1 to 5 as a surfactant or foam booster in cleaners and cosmetic products.

## Revendications

1. Copolymères dérivés de poly(acide aspartique)s, qui sont constitués, à raison d'au moins 75 % des motifs présents, de motifs structuraux de formules générales (I) et (II) 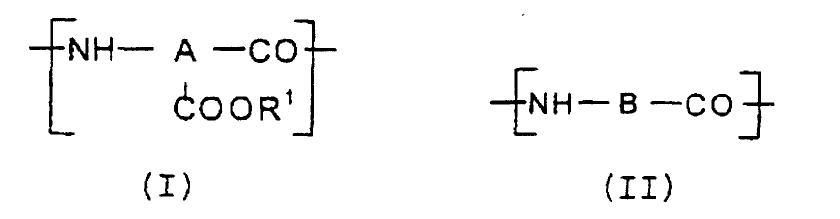 dans lesquelles l'élément structural A consiste en des radicaux hydrocarbonés trifonctionnels identiques ou différents ayant 2 atomes de carbone, de structure (A1) ou (A2) 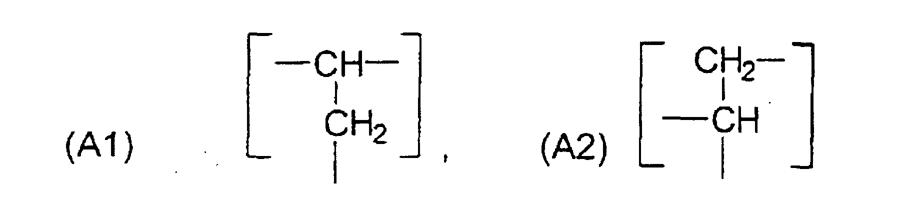 un copolymère consistant en au moins trois motifs de formule (I), dans laquelle
R¹ peut avoir la signification de R², R³ ou R⁴,
R² représente un ou plusieurs radicaux choisis dans le groupe constitué par les métaux alcalino, alcalino-terreux, l'hydrogène ou l'ammonium, [NR⁵R⁶R⁷R⁸]⁺, R⁵ à R⁸ représentant, indépendamment les uns des autres, un atome d'hydrogène ou un groupe alkyle ou hydroxyalkyle,
R³ représente des radicaux alkyle R⁹ identiques ou différents, à chaîne droite ou ramifiés, saturés ou insaturés, ayant de 6 à 24 atomes de carbone, ou des radicaux de structure -X-R⁹, X étant une chaîne polyoxyalkylène comportant de 1 à 100 unités oxyalkylène,
R⁴ représente des radicaux alkyle identiques ou différents, à chaîne droite ou ramifiés, saturés ou insaturés, ayant de 1 à 5 atomes de carbone,
et dans chaque cas au moins un radical R¹ doit adopter la signification de R² et au moins un radical R¹ doit adopter celle de R³ ou R⁴, et
les motifs [-NH-B-CO] sont des composants choisis dans le groupe des aminoacides protéinogènes ou non protéinogènes et sont contenus à raison d'au maximum 20 % en poids.

2. Copolymères selon la revendication 1, dans lesquels au moins un radical R¹ a la signification de R² et au moins un radical R¹ a la signification de R⁴.

3. Copolymères selon la revendication 1, dans lesquels au moins un radical R¹ a la signification de R² et au moins un radical R¹ a la signification de R³.

4. Copolymères selon la revendication 1, dans lesquels au moins un radical R¹ a la signification de R², au moins un radical R¹ a la signification de R³ et au moins un radical R¹ a la signification de R⁴.

5. Copolymères selon les revendications 1, 3 et 4, dans lesquels R³ représente des radicaux alkyle R¹⁰ identiques ou différents, à chaîne droite ou ramifiés, saturés ou insaturés, ayant de 8 à 24 atomes de carbone.

6. Procédé pour la préparation des copolymères selon la revendication 1, **caractérisé en ce qu'**on fait réagir partiellement un poly(imide d'acide aspartique), dans une première étape, avec des alcools R⁴OH et éventuellement, dans une deuxième étape, avec des alcools de structure R³OH ou R³-X-OH, R⁴, R³ et X ayant les significations données plus haut, et, dans une troisième étape éventuellement subséquente, les groupes ester dérivés de R⁴OH sont partiellement ou totalement hydrolysés dans des conditions douces.

7. Procédé pour la préparation des copolymères selon l'une quelconque des revendications 1 à 5, **caractérisé en ce qu'**on fait réagir avec de l'ammoniac et polymérise des esters d'acides dicarboxyliques α,β-insaturés ou leurs sels d'ammonium, de formules générales (III) et (IV) 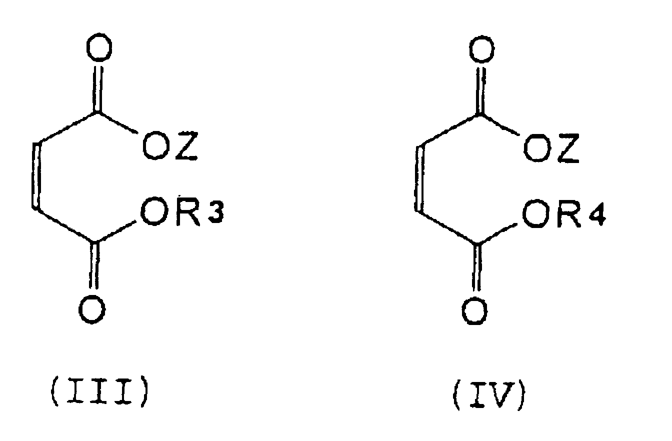 seuls ou en mélange entre eux, formules dans lesquelles Z représente un atome d'hydrogène et/ou l'ion ammonium, et R³ et R⁴ représentent les radicaux indiqués plus haut, éventuellement en présence de jusqu'à 20 % en poids d'aminoacides protéinogènes ou non protéinogènes ou de leurs dérivés, et éventuellement, dans d'autres étapes, on engendre par hydrolyse des groupes de structure de la structure de formule (I), dans laquelle R¹ a la signification de R², avec la définition de R² indiquée plus haut.

8. Procédé selon la revendication 7, **caractérisé en ce qu'**on utilise comme esters des acides dicarboxyliques α,β-insaturés ou leurs sels d'ammonium et des dérivés d'acide maléique.

9. Utilisation des copolymères selon les revendications 1 à 5 dans des préparations contenant des tensioactifs.

10. Utilisation des copolymères selon les revendications 1 à 5, en tant qu'émulsifiant, renforçateur de mousse, adjuvant de détergent, complexant pour cations polyvalents, adjuvant de dispersion ou conditionneur.

11. Utilisation des copolymères selon les revendications 1 à 5, en tant que tensioactif ou renforçateur de mousse dans des produits de nettoyage et des préparations cosmétiques.
